(19) Europäisches Patentamt
European Patent Office
Office européen des brevets

(11) Publication number: **0 015 767**
**B1**

(12) # EUROPEAN PATENT SPECIFICATION

(45) Date of publication of patent specification: **11.05.83**

(51) Int. Cl.³: **C 12 Q 1/28**, C 12 Q 1/54, C 12 Q 1/60, C 12 Q 1/62, G 01 N 33/54

(21) Application number: **80300714.5**

(22) Date of filing: **07.03.80**

(54) **Compositions, methods and elements for detecting or assaying peroxidatively active substances.**

(30) Priority: **08.03.79 US 18532**

(43) Date of publication of application:
**17.09.80 Bulletin 80/19**

(45) Publication of the grant of the patent:
**11.05.83 Bulletin 83/19**

(84) Designated Contracting States:
**DE FR GB**

(56) References cited:
**US - A - 3 654 090**
**US - A - 3 992 158**
**US - A - 4 042 335**

**CHEMICAL ABSTRACTS, vol. 90, no. 16, 16 April 1979, abstract no. 134675r, page 236, Columbus, Ohio, US, T. KATO et al.: "Stabilization of dichlorofluorescin and its application to the determination of biological substances using oxidase"**

(73) Proprietor: **EASTMAN KODAK COMPANY**
**343 State Street**
**Rochester, New York 14650 (US)**

(72) Inventor: **Frank, David Stanley**
**Kodak Park**
**Rochester, New York (US)**

(74) Representative: **Pepper, John Herbert et al,**
**KODAK LIMITED Patent Department P.O. Box 114**
**190 High Holborn**
**London WC1V 7EA (GB)**

(56) References cited:
**CHEMICAL ABSTRACTS, vol. 91, no. 11, 10th September 1979, abstract no. 86926v, page 383, Columbus, Ohio, US**

**BULL. SOC. CHIM. BIOL., 1968, vol. 50, no. 5, 6, A. STRATIS: "Détection d'anticorps et d'antigènes a l'aide d'enzymes" pages 1169—78.**

**EP 0 015 767 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Compositions, methods and elements for detecting or assaying peroxidatively active substances

This invention relates to compositions and elements for detecting or assaying substances having peroxidative activity and to methods of assaying therewith.

The oxidation of dichlorofluorescin by hydrogen peroxide to dichlorofluorescein, catalyzed by the enzyme peroxidase, has been used as an assay technique for hydrogen peroxide (Black, M.J. & Brandt, R.B., Anal, Biochem, 58:246—254 [1974]). A drawback of this technique is that since the dichlorofluorescin is unstable to air oxidation, it must be generated *as needed* by hydrolysis of diacetyldichlorofluorescin at alkaline pH followed by neutralization and dilution in the assay tube.

Chemical Abstracts, vol. 90, No. 16, 16 April 1979, abstract No. 134,675r describes a method of stabilising diacetyldichlorofluorescin in the determination of uric acid using cycloheptaamylase. The reactants are incubated at 37° for 20 minutes after which the fluorescence emission is measured. From the concentration ranges disclosed it is calculated that the mole ratio of diacetyldichlorofluorescin to uric acid ranges from 12.9:1 to 1.03:1.

The assay of peroxidase, particularly at relatively low levels, is useful in certain immunoassay techniques wherein a peroxidase is used as the label for a ligand in a competitive binding or displacement immunological determination. Such assay techniques are described in U.S. Patent 3,817,837; U.S. Patent 3,654,090 and in Van Weeman and Schuurs, BIOCHIMIE, *54*:842 (1972).

There is a need for a peroxidase assay which is capable of detecting low levels of the enzyme, i.e., on the order of $\leq 10^{-7}$ M, as are used in immunoassays. Such as assay should be rapid and eliminate the requirement for the dichlorofluorescin being prepared as needed in a separate step. Furthermore, it is most desirable that all of the reagents can be stored in a stable, dry condition until immediately prior to use.

We now provide such a rapid and sensitive method for the assay of a substance having peroxidative activity and compositions for use therein.

According to the present invention there is provided a composition for the detection or assay of a substance having peroxidative activity, said composition characterised in that it contains diacetyldichlorofluorescin and a source of hydrogen peroxide, the mole ratio of diacetyldichlorofluorescin to hydrogen peroxide or generatable hydrogen peroxide ranging from 1:5 to 1:2500.

The present invention also provides a method for the detection or assay of substances demonstrating peroxidative activity which comprises the steps of 1) contacting an assay composition according to the present invention with a sample for analysis to produce dichlorofluorescein and 2) detecting the resulting dichlorofluorescein.

In a preferred embodiment the rate of change of fluorescence of dichlorofluorescein is detected. As long as all reactants other than the peroxidase are present in excess, the rate of change of fluorescence can be linearly related to the amount of peroxidase in the sample under assay.

The present invention also provides dry reagent compositions that include all of the materials required to perform a peroxidase determination. Such compositions can be stored dry and provide stable and adaptable compositions for a multitude of applications wherein peroxidase detection and/or quantification may be useful or desirable.

The source of hydrogen peroxide can be hydrogen peroxide itself or a composition which generates hydrogen peroxide e.g., glucose and glucose oxidase or uric acid and uricase. Preferably the present compositions contain a buffer to maintain the pH of the assay composition at between 7 and 9.

A further embodiment of the present invention comprises an element for the detection of peroxidase comprising a spreading layer and a reagent layer in fluid contact under conditions of use, said reagent layer containing an assay composition according to the present invention.

Particular advantages of the peroxidase assay method described herein are:

(1) Simplicity: dichlorofluorescin is generated *in situ* during the assay reaction thus eliminating costly preparation steps;

(2) Reagent Stability: since all required reagents are capable of dry storage, the assay composition exhibits extended shelf life and can be adapted to numerous assay applications;

(3) Speed: the assay compositions are capable of assaying for peroxidase activity in one minute or less.

Peroxidative activity is well known in the art and includes, for example, peroxidative enzymes, cytochromes and haemoglobin. Thus, the assay procedure discussed herein is useful not only in the assay of what are technically termed peroxidase enzymes, but also in the determination of other materials demonstrating "peroxidative activity."

The reaction which occurs in one of the preferred assay compositions described herein is as follows:

$$\text{Gluconic Acid} + H_2O_2 \xrightleftharpoons{\text{Glucose Oxidase}} \text{Gluconic Acid}$$

$$H_2O_2 + \text{Diacetyldichlorofluorescin} \rightarrow \text{Dichlorofluorescin} + 2\ \text{HOAc}$$

$$\text{Dichlorofluorescin} + H_2O_2 \xrightleftharpoons{\text{Peroxidase}} \text{Dichlorofluorescein (Fluorescent)}$$

Diacetyldichlorofluorescin is readily and commercially available from Eastman Organic Chemicals, Rochester, New York.

Any source of hydrogen peroxide can be used in the successful practice of the present invention. Of course, hydrogen peroxide itself can be used as the source of the material. This is preferred when the assay reaction is carried out in solution or when the hydrogen peroxide can be added to the reagent system at about the same time as the sample under assay.

In many circumstances, it may, however, be desirable to supply a reagent composition of the type described herein in dry powder form ready for reconstitution with water or to provide the assay composition in the form of a paper or other fibrous or strip material, e.g. a dip-and-read strip, which has been impregnated or coated with reagent which is reconstituted upon application of a liquid assay sample thereto. Yet a further desirable embodiment can be the incorporation of the reagent into one or more layers of a multilayer element of the type described in U.S. Patent 3,992,158.

Under such circumstances, a source of hydrogen peroxide that is dry until contacted with water should be used. Preferred sources are mixtures of an enzyme that demonstrates oxidative activity and substrates for such an enzyme, for example, glucose and glucose oxidase, uric acid and uricase, and cholesterol and cholesterol oxidase. Any of these and numerous other similar mixtures of oxidase and substrate when contacted in water and in the presence of oxygen result in decomposition of the substrate with the concomitant production of hydrogen peroxide. Such materials are particularly useful because they can be freeze-dried or lyophilized to provide dried powders that can be reconstituted with water. The enzyme and the substrate should preferably be kept separated until the production of the hydrogen peroxide is desired.

Furthermore, since dichlorofluorescin is unstable, its formation by the reaction of peroxide and diacetyldichlorofluorescin should not be initiated until the substance under assay having peroxidative activity has been contacted with the assay composition. Other useful hydrogen peroxide sources include chemically bound hydrogen peroxides, such as urea peroxide (Robeco Chemicals, Inc.).

The amount of the reagents in the assay composition will depend, to a large extent, on the amount of the material under assay — higher amounts of reagent being necessary for higher amounts of material under assay. For concentrations of substance having peroxidative activity between $10^{-5}$ and $10^{-10}$ M, a solution having the following composition has been found particularly useful:

| | |
|---|---|
| diacetyldichlorofluorescin | $10^{-7}$—$10^{-4}$ M |
| oxidative enzyme | 1—50 units/ml |
| substrate | 1—100 mg/dl |
| Buffer to maintain pH at | 7—9. |

When the composition is in the form of a solution the concentration of diacetyldichlorofluorescin is preferably from $10^{-7}$ to $10^{-3}$ M, and the concentration of the source of hydrogen peroxide is from $10^{-6}$ ro $10^{-2}$ M.

To be useful, a buffer should not interfere with the assay reaction. Barbital or phosphate buffers at a concentration of between about 0.01 and 0.1 M have been found useful.

When hydrogen peroxide is used as the peroxide source, a concentration of between $10^{-5}$ and $5 \times 10^{-4}$ M has been found useful in the above-described assay composition.

In a further embodiment of the present invention, the assaying composition comprising diacetyldichlorofluorescin and a source of hydrogen peroxide can be used to determine the amount of peroxidative activity present in a patient's haemoglobin. Thus, a sample of haemoglobin can be added to the assaying composition and the measurement of the fluorescence rate of change of the resulting dichlorofluorescein will determine the amount of peroxidative activity in the haemoglobin.

The assay composition can be employed in dry chemistry techniques as mentioned above, for example in the dry test elements described in U.S. Patents 3,992,158 and 4,042,335.

The element can comprise zones of reagent, which zones may be in the form of layers, and, optionally, spreading agent. Elements containing these zones are described in U.S. Patents 3,992,158 and 4,069,016.

The substantially dry element can be contacted with a sample for analysis and the resulting dichlorofluorescein can be detected. If the sample itself does not contain water, it could be added with the sample to the dry element.

A particularly preferred test element for the detection of a substance having peroxidative activity, e.g. peroxidase, in a liquid comprises a spreading layer, a reagent layer and a registration layer, all in fluid contact under conditions of use, and a support (preferably transparent), the reagent layer intervening the spreading layer and the registration layer and the registration layer intervening the reagent layer and the support, the reagent layer containing a composition according to the present invention. Such registration layers are described in detail in U.S. Patent 4,042,355, columns 11 and 12.

In a still further embodiment of the present invention, a method of assaying for antigen or antibody present in a sample comprises:

a) labelling a known amount of antigen or antibody with a substance having peroxidative activity and adding the labelled antigen or antibody with the sample to be assayed to a known amount of corresponding antibody or antigen respectively; and

b) determining the amount of unknown antigen or antibody by

1) contacting the mixture of (a) and a composition according to the present invention to produce dichlorofluorescein and

2) detecting the dichlorofluorescein.

The above immunoassay may be carried out in the conventional manner. Thus, the unknown sample can be added along with the labelled sample to a liquid or web comprising the corresponding antibody (or antigen). The labelled and unlabelled antigen (or antibody) will combine with the corresponding antibody (or antigen) to form an antigen-antibody precipitate and free labelled antigen (or antibody) as well as free unlabelled antigen (or antibody). The determination of the unknown antibody (or antigen) is made by measuring the free labelled antigen (or antibody) or the labelled antigen-antibody precipitate.

The measurement of labelled antigen or antibody can be accomplished by assaying for the substance having peroxidative activity by adding the resulting solution to an assaying composition comprising diacetyldichlorofluorescin and a source of hydrogen peroxide and determining the amount of substance having peroxidative activity by measuring the rate of change of fluorescence of the resulting dichlorofluorescein.

The following Examples demonstrate the successful practice of the present invention.

## Example 1

### Fluorescence Assay for Peroxidase

To a 1 $cm^2$ cuvette was added 1.4 ml phosphate buffer solution (PBS) containing 0.01 M sodium phosphate, 0.1 N NaCl, pH 7.5, 0.200 ml diacetyldichlorofluorescin ($1 \times 10^{-5}$ M in PBS) and 200 $\mu$l hydrogen peroxide ($1 \times 10^{-3}$ M in water). Reaction was initiated by the addition of peroxidase (horseradish) of the indicated concentration. The rate of fluorescence increase was measured in a Farrand Mk I fluorometer with excitation monochrometer at 490 nm and emission monochrometer at 520 nm. The rate was linear for 5 minutes. In the Figure of the accompanying drawings, the rates of fluorescence increase per minute are plotted as a function of peroxidase concentration.

Peroxidase concentrations of up to $1.32 \times 10^{-5}$ M were measurable when the hydrogen peroxide concentration was increased fifty fold and the dichlorofluorescin concentration increased twofold.

## Example 2

### Multilayered Element for the Fluorometric Determination of Peroxidase

A multilayered element was prepared according to the following:

A polycarbonate support was coated with a reagent layer comprised of deionized gelatin (Type V) (4.5 $g/m^2$), 5,5-dimethyl-1,3-cyclohexanedione (0.4 $g/m^2$), glucose oxidase (538 Units/$m^2$), bis(vinyl sulphonyl methyl)ether (0.05 $g/m^2$), Surfactant 10G, a nonylphenoxypolyglycidol from Olin Corp. (0.01 $g/m^2$), zinc acetate (0.01 $g/m^2$), dichlorofluorescin diacetate (0.01 $g/m^2$) in 0.02 M phosphate buffer at pH 8.0; an enzyme layer comprising deionized gelatin (2.9 $g/m^2$), colloidal silver (1.02 $g/m^2$), catalase (86,000 U/$m^2$), bis(vinyl sulphonyl methyl) ether (0.04 $g/m^2$), Surfactant 10G (0.007 $g/m^2$); a subbing layer comprised of poly-N-isopropylacrylamide (0.4 $g/m^2$); and a spreading layer comprised of $TiO_2$ (70.0 $g/m^2$), cellulose acetate (10.0 $g/m^2$), glucose (0.5 $g/m^2$), and polyacrylamide particles (L60 mesh) (0.01 $g/m^2$.

The element was tested by applying thereto a 10 $\mu$l drop of solution containing dinitrophenyl peroxidase conjugate prepared by coupling dinitrobenzene sulphonic acid to peroxidase obtained from horseradish (DNP.POD) and measuring the rate at which the fluorescent signal increased, when excited at 460 nm and emitted at 520 nm, as in Example 1. Results are shown in Table I.

TABLE I

| Conc. DNP.POD (M) | Relative Rate |
|---|---|
| $5.0 \times 10^{-6}$ | 7.5 |
| $2.5 \times 10^{-6}$ | 4.8 |
| $1.25 \times 10^{-6}$ | 1.92 |
| $6.25 \times 10^{-7}$ | 0.79 |
| $3.13 \times 10^{-7}$ | 0.27 |
| $1.57 \times 10^{-7}$ | 0.09 |
| $7.85 \times 10^{-8}$ | 0.05 |
| $3.95 \times 10^{-8}$ | 0.028 |
| 0 | 0 |

## Claims

1. A composition for the detection or assay of a substance having peroxidative activity, said

composition characterized in that it contains diacetyldichlorofluorescin and a source of hydrogen peroxide, the mole ratio of diacetyldichlorofluorescin to hydrogen peroxide or generatable hydrogen peroxide ranging from 1:5 to 1:2500.

2. The composition of claim 1 wherein the source of hydrogen peroxide is:

A. an enzyme and an enzyme substrate which, when contacted in an aqueous medium in the presence of oxygen, release hydrogen peroxide;

B. hydrogen peroxide, per se; or

C. urea peroxide.

3. The composition of claim 2 wherein an enzyme and an enzyme substrate are employed, the enzyme being an oxidative enzyme and the substrate being the corresponding substrate for the oxidative enzyme.

4. The composition of claim 3 wherein the oxidative enzyme and corresponding substrate are:

A. glucose oxidase and glucose,

B. uricase and uric acid, or

C. chlorestrol oxidase and cholesterol.

5. The composition of any of the preceding claims further including a buffer for maintaining the pH between 7 and 9.

6. The composition of any of the preceding claims which is in the form of a solution wherein the concentration of diacetyldichlorofluorescin is from $10^{-7}$ to $10^{-3}$ M, and the concentration of the source of hydrogen peroxide is from $10^{-6}$ to $10^{-2}$ M.

7. A method for detecting or assaying a substance having peroxidative activity characterised by the steps of:

(1) contacting a sample for analysis and the composition of any of claims 1—6 to produce dichlorofluoresein; and

(2) detecting the dichlorofluorescein.

8. The method of claim 7 in which a substantially dry element comprising a reagent zone containing the composition of any of claims 1—6 is contacted with a liquid sample for analysis.

9. The method of claim 7 or 8 wherein the dichlorofluorescein is detected by fluorescence rate of change measurements.

10. A dry test element for the detection or assay of a substance having peroxidative activity in an aqueous liquid characterised in that said element has a reagent zone containing the assay composition of any of claims 1—6.

11. An element of claim 10 comprising fibrous material impregnated with the composition of any of claims 1—6.

12. The element of claim 11 wherein the fibrous material is paper.

13. An element of claim 12 comprising a spreading layer and a reagent layer in fluid contact under conditions of use, said reagent layer containing the composition of any of claims 1—6.

14. An element of claim 13 comprising the spreading layer, the reagent layer, and a registration layer all in fluid contact under conditions of use and a support, the reagnet layer intervening the spreading layer and the registration layer, and the registration layer intervening the reagent layer and the support.

15. A method of assaying for antigen or antibody present in a sample to be tested characterized by:

(a) labelling a known amount of antigen or antibody with a substance having peroxidative activity and adding said labelled antigen or antibody with said sample to be tested to a known amount of corresponding antibody or antigen respectively; and

(b) determining the amount of unknown antigen or antibody by

(1) contacting the mixture of (a) and the composition of any of claims 1—6 in the presence of water to produce dichlorofluorescein, and

(2) detecting the dichlorofluorescein.

**Revendications**

1. Composition pour la détection ou la détermination quantitative d'une substance possédant une activité peroxydante, cette composition étant caractérisée en ce qu'elle contient de la diacétyldichlorofluorescine et une source de peroxyde d'hydrogène, le rapport molaire de la diacétyldichlorofluorescine au peroxyde d'hydrogène, ou au peroxyde d'hydrogène susceptible d'être formé, étant compris entre 1:5 et 1:2500.

2. Composition conforme à la revendication 1, dans laquelle la source de peroxyde d'hydrogène est choisie dans le groupe constitué par (A) une enzyme et un substrat d'enzyme qui, au contact d'un milieu aqueux, en présence d'oxygène, libèrent du peroxyde d'hydrogène, (B) le peroxyde d'hydrogène, luimême, et, (C) le peroxyde d'urée.

3. Composition, conforme à la revendication 2, dans laquelle sont présents une enzyme et un substrat d'enzyme, l'enzyme étant une enzyme oxydante et le substrat étant le substrat correspondant à l'enzyme oxydante.

4. Composition, conforme à la revendication 3, dans laquelle l'enzyme oxydante et son substrat correspondant sont choisis dans les groupes constitués par (A) la glucose-oxydase et le glucose, (B) l'uricase et l'acide urique, et, (C) la cholestérol-oxydase et le cholestérol.

5. Composition, conforme à l'une quelconque des revendications 1 à 4, qui comprend, en outre, un agent tampon pour maintenir le pH entre 7 et 9.

6. Composition conforme à l'une quelconque des revendications 1 à 5, qui se présente sous la forme d'une solution dans laquelle la concentration en diacétyl-dichlorofluorescine est comprise entre $10^{-7}$ M et $10^{-3}$ M et la concentration en source de péroxyde d'hydrogène est comprise entre $10^{-6}$ M

et $10^{-2}$ M.

7. Procédé pour la détection ou la détermination quantitative d'une substance possédant une activité peroxydante, carctérisé en ce qu'il comprend les étapes suivantes:

(1) on traite un échantillon à analyser au contact d'une composition, conforme à l'une quelconque des revendications 1 à 6, pour former de la dichlorofluorescéine, et,

(2) on détecte ou on détermine la dichlorofluorescéine.

8. Procédé, conforme à la revendication 7, par lequel on amène au contact d'un produit, pratiquement sec, qui comprend une zone de réactif qui contient une composition conforme à l'une quelconque des revendications 1 à 6, un échantillon liquide du produit à analyser.

9. Procédé conforme à la revendication 7 ou 8, par lequel on détecte ou on détermine la dichlorofluorescéine par la mesure de la vitesse de la variation de la fluorescence.

10. Produit, à l'état sec, pour la détection ou la détermination d'une substance possédant une activité peroxydante dans un liquide aqueux, caractérisé en ce que ce produit comprend une zone de réactif qui contient une composition conforme à l'une quelconque des revendications 1 à 6.

11. Produit conforme à la revendication 10, qui comprend une substance fibreuse imprégnée d'une composition conforme à l'une quelconque des revendications 1 à 6.

12. Produit conforme à la revendication 11, dont la substance fibreuse est constituée par du papier.

13. Produit conforme à la revendication 12, qui comprend une couche d'étalement et une couche de réactif, en contact fluide dans les conditions d'utilisation, cette couche de réactif contenant une composition conforme à l'une quelconque des revendications 1 à 6.

14. Produit conforme à la revendication 13, qui comprend, outre un support, la dite couche d'étalement et la dite couche de réactif ainsi qu'une couche d'enregistrement, toutes ces couches étant en contact fluide dans les conditions d'utilisation, la couche de réactif reliant la couche d'étalement et la couche d'enregistrement, et la couche d'enregistrement reliant la couche de réactif et le support.

15. Procédé pour la détermination d'antigène ou d'anticorps présents dans un échantillon d'essai, caractérisé en ce que (a) on marque une quantité déterminée d'antigène ou d'anticorps au moyen d'une substance présentant une activité peroxydante et on ajoute cet antigène ou anticorps ainsi marqué avec l'échantillon d'essai à une quantité connue respectivement de l'anticorps ou de l'antigène correspondant, et, (b) on détermine la quantité d'antigène ou d'anticorps inconnue par les opérations qui comprennent (1) le contact d'une mélange défini sous (a) et d'une composition conforme à l'une quelconque des revendications 1 à 6, en présence d'eau, pour former la dichlorofluorescéine et (2) la détection de cette dichlorofluorescéine.

**Patentansprüche**

1. Mittel für den Nachweis oder die Bestimmung einer Substanz mit peroxidativer Aktivität, dadurch gekennzeichnet, daß es Diacetyldichlorfluoreszein und einen Wasserstoffperoxidlieferanten enthält, wobei das Molverhältnis von Diacetyldichlorfluoreszein zu Wasserstoffperoxid oder generierbarem Wasserstoffperoxid im Bereich von 1:5 bis 1:2500 liegt.

2. Mittel nach Anspruch 1, dadurch gekennzeichnet, daß der Wasserstoffperoxidlieferant ist:

A. ein Enzym und ein Enzymsubstrat, die, wenn sie in einem wäßrigen Medium in Gegenwart von Sauerstoff miteinander in Kontakt gebracht werden, Wasserstoffperoxid freisetzen;

B. Wasserstoffperoxid per se oder

C. Harnstoffperoxid.

3. Mittel nach Anspruch 2, das ein Enzym und ein Enzymsubstrat einthält, dadurch gekennzeichnet, daß das Enzym ein oxidatives Enzym ist und daß das Substrat das entsprechende Substrat für das oxidative Enzym ist.

4. Mittel nach Anspruch 3, dadurch gekennzeichnet, daß das oxidative Enzym und das entsprechende Substrat ist:

A. Glucoseoxidase und Glucose,

B. Uricase und Harnsäure oder

C. Cholesterinoxidase und Cholesterin.

5. Mittel nach einem der vorstehenden Ansprüche, dadurch gekennzeichnet, daß es einen Puffer für die Aufrechterhaltung des pH-Wertes zwischen 7 und 9 enthält.

6. Mittel nach einem der vorstehenden Ansprüche, dadurch gekennzeichnet, daß es in Form einer Lösung vorliegt, in der die Konzentration des Diacetyldichlorfluoreszeins bei $10^{-7}$ bis $10^{-3}$ M und die Konzentration des Wasserstoffperoxidlieferanten bei $10^{-6}$ bis $10^{-2}$ M liegt.

7. Verfahren zum Nachweis oder zur Bestimmung einer Substanz mit peroxidativer Aktivität, gekennzeichnet durch die folgenden Stufen:

(1) Inkontaktbringen einer Analyseprobe mit dem Mittel gemäß einem der Ansprüche 1 bis 6 zum Zwecke der Erzeugung von Dichlorfluoreszein und

(2) Nachweis des Dichlorfluoreszeins.

8. Verfahren nach Anspruch 7, dadurch gekennzeichnet, daß ein im wesentlichen trockenes Element mit einer Reagenszone mit dem Mittel nach einem der Ansprüche 1 bis 6 mit einer flüssigen, zu analysierenden Probe in Kontakt gebracht wird.

9. Verfahren nach Anspruch 7 oder 8, dadurch gekennzeichnet, daß das Fluoreszein durch Messungen der Veränderung des Fluoreszenzgrades bestimmt wird.

10. Trockenes Testelement für den Nachweis oder die Bestimmung einer Substanz mit peroxidativer Aktivität in einer wäßrigen Flüssigkeit, dadurch gekennzeichnet, daß das Element eine Reagenszone mit dem Nachweismittel nach einem der Ansprüche 1 bis 6 aufweist.

11. Element nach Anspruch 10, gekennzeichnet, durch eine faseriges, mit dem Mittel nach einem der Ansprüche 1 bis 6 imprägniertes Material.

12. Element nach Anspruch 11, dadurch gekennzeichnet, daß das faserige Material Papier ist.

13. Element nach Anspruch 12, dadurch gekennzeichnet, daß es eine Ausbreitschicht und eine Reagensschicht aufweist, die sich unter den Gebrauchsbedingungen des Elementes im Strömungsoder Flüssigkeitskontakt miteinander befinden, und daß die Reagensschicht das Mittel nach einem der Ansprüche 1 bis 6 enthält.

14. Element nach Anspruch 13, dadurch gekennzeichnet, daß es die Ausbreitschicht, die Reagensschicht und eine Registrierschicht, die sich unter den Gebrauchsbedingungen des Elementes im Strömungs- oder Flüssigkeitskontakt miteinander befinden, und einen Träger aufweist und daß die Reagensschicht zwischen der Ausbreitschicht und der Registrierschicht und die Registrierschicht zwischen Reagensschicht und Träger angeordnet sind.

15. Verfahren zur Bestimmung eines Antigens oder eines Antikörpers in einer zu testenden Probe, gekennzeichnet durch:

(a) Kennzeichnung oder Markierung einer bekannten Menge eines Antigens oder Antikörpers mit einer Substanz mit peroxidativer Aktivität und Zufügen des gekennzeichneten bzw. markierten Antigens oder Antikörpers mit der zu untersuchenden Probe zu einer bekannten Menge des entsprechenden Antikörpers bzw. Antigens und

(b) Bestimmung der Menge an unbekanntem Antigen oder Antikörper durch

(1) Inkontaktbringen der Mischung von (a) und dem Mittel nach einem der Ansprüche 1 bis 6 in Gegenwart von Wasser zum Zwecke der Erzeugung von Dichlorfluoreszein und

(2) Bestimmung des Dichlorfluoreszeins.

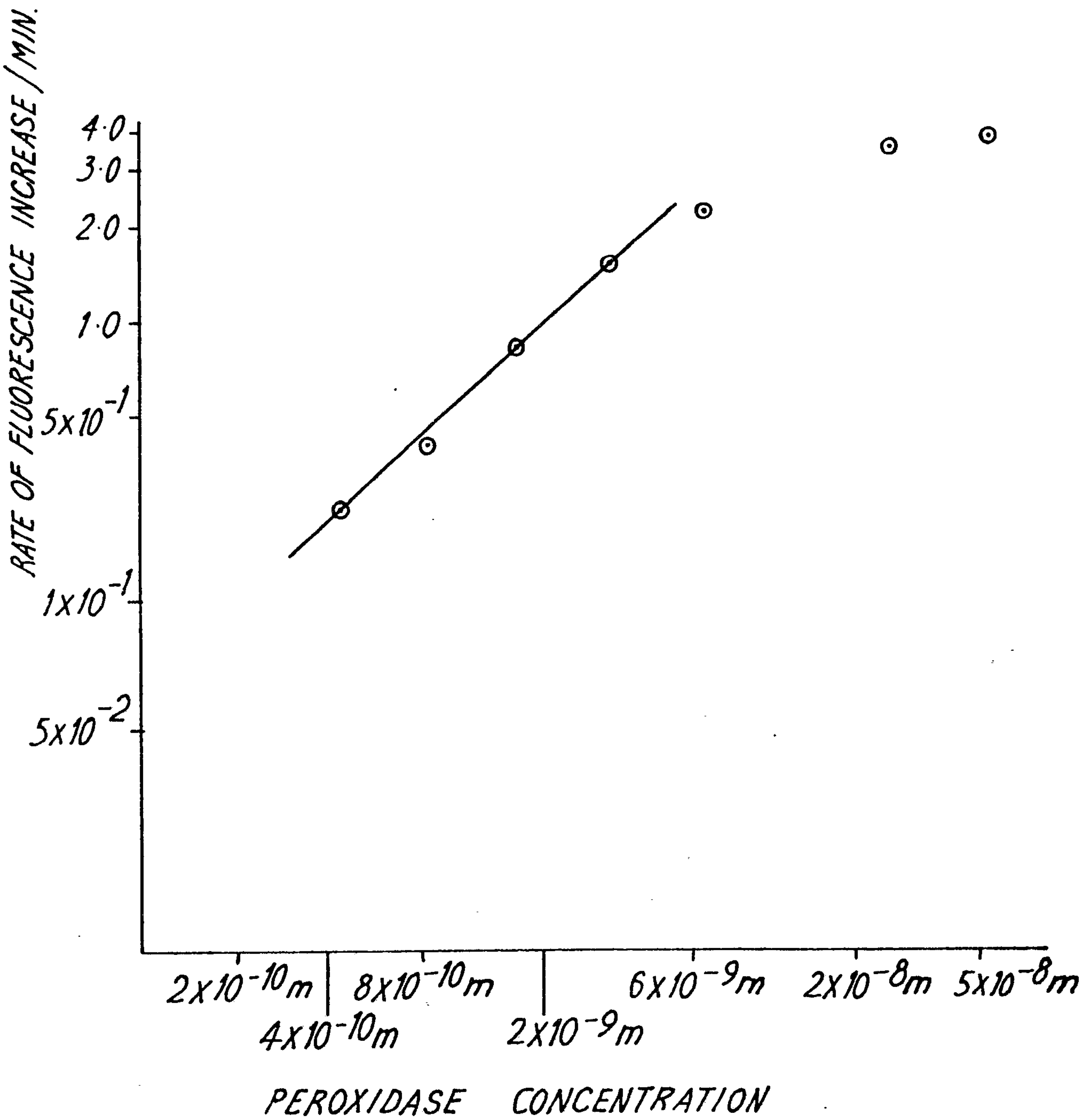
RATE OF FLUORESCENCE INCREASE / MIN.
4·0
3·0
2·0
1·0
5x10^-1
1x10^-1
5x10^-2
2x10^-10m
4x10^-10m
8x10^-10m
2x10^-9m
6x10^-9m
2x10^-8m
5x10^-8m
PEROXIDASE CONCENTRATION